(19) Europäisches Patentamt
European Patent Office
Office européen des brevets

(11) **EP 2 939 631 A1**

(12) **EUROPEAN PATENT APPLICATION**

(43) Date of publication:
**04.11.2015 Bulletin 2015/45**

(51) Int Cl.:
**A61B 18/26** (2006.01)  **A61B 18/24** (2006.01)

(21) Application number: **15166049.5**

(22) Date of filing: **30.04.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **30.04.2014 US 201461986446 P**

(71) Applicant: **Cook Medical Technologies LLC Bloomington, IN 47404 (US)**

(72) Inventors:
• **Milner, Thomas**
  **Austin, TX Texas 78712 (US)**
• **Teichman, Joel**
  **Vancouver, British Columbia V6T 1Z4 (CA)**

(74) Representative: **Garratt, Peter Douglas et al**
  **Mathys & Squire LLP**
  **The Shard**
  **32 London Bridge Street**
  **London SE1 9SG (GB)**

(54) **LASER APPARATUS FOR CALCULI REMOVAL**

(57)    A set of ideal values for laser parameters is provided for a laser system for the fragmentation and removal of calculi. Particularly, the ideal values for laser parameters increases the effectiveness of removing calculi by increasing the pulse width. This set of ideal values for laser parameters increases the effectiveness of calculi removal by decreasing retropulsion distance of calculi fragments and by breaking up the calculi into smaller fragments that can then be easily removed from the body without the use of baskets or graspers.

(Figure 1)

## Description

### TECHNICAL FIELD

This disclosure generally relates to the use of a laser system for the removal of unwanted materials such as calculi, deposits and tissue from body lumens. More particularly, this disclosure relates to ideal values for laser parameters used in a laser system to remove calculi from the body.

### BRIEF SUMMARY

Disclosed is a set of ideal values for laser parameters for use in a pulsed laser system for the fragmentation and removal of calculi. Particularly, the disclosed set of ideal values for laser parameters are for use in a laser system that uses a Ho:YAG laser. Particularly, the ideal values for laser parameters minimizes the amount of calculi movement while providing for calculi fragmentation by increasing the pulse width. Doing so reduces retropulsion distance that can cause trauma to the surrounding tissue and allows for the removal of the fragmented calculi through the voiding of the water or saline flow used during the procedure. The disclosed system can also be used on soft tissue procedures to remove polyps or tumor cells.

In one embodiment, an apparatus for fragmenting calculi comprises a source of laser pulses, an optical fiber having a distal end configured to be in close proximity with said calculi and a proximal end that is configured to receive laser pulses from said source of laser pulses when said optical fiber is operatively engaged with said source of laser pulses, and a source of laser pulses is configured to specifically generate laser pulses with an optical pulse width between 400 $\mu$s and 600 $\mu$s.

In another embodiment, the apparatus for fragmenting calculi comprises a source of laser pulses configured to specifically generate laser pulses with an optical pulse width between 600 $\mu$s and 1000 $\mu$s.

In another embodiment, the apparatus for fragmenting calculi comprises a source of laser pulses configured to specifically generate laser pulses with an optical pulse width of 438.8 $\mu$s.

In another embodiment, the apparatus for fragmenting calculi comprises a source of laser pulses configured to specifically generate laser pulses with an optical pulse width of 584.4 $\mu$s.

In another embodiment, the apparatus for fragmenting calculi comprises a source of laser pulses configured to specifically generate laser pulses with an electrical pulse width at least 1000 $\mu$s.

In another embodiment, the apparatus for fragmenting calculi comprises a source of laser pulses configured to specifically generate laser pulses with an electrical pulse width of 1000 $\mu$s.

In another embodiment, the apparatus for fragmenting calculi comprises a source of laser pulses configured to specifically generate laser pulses with an electrical pulse width of 1250 $\mu$s.

In another embodiment, a method for fragmenting calculi comprises providing a source of laser pulses, providing an optical fiber having a distal end configured to be in close proximity with the calculi and a proximal end that is configured to receive laser pulses from the source of laser pulses when the optical fiber is operatively engaged with the source of laser pulses, and calibrating the source of laser pulses to specifically generate laser pulses with an optical pulse width between 400 $\mu$s and 600 $\mu$s.

In another embodiment, the method for fragmenting calculi comprises a source of laser pulses configured to specifically generate laser pulses with an optical pulse width between 600 $\mu$s and 1000 $\mu$s.

In another embodiment, the method for fragmenting calculi comprises a source of laser pulses configured to specifically generate laser pulses with an optical pulse width of 438.8 $\mu$s.

In another embodiment, the method for fragmenting calculi comprises a source of laser pulses configured to specifically generate laser pulses with an optical pulse width of 584.4 $\mu$s.

In another embodiment, the method for fragmenting calculi comprises a source of laser pulses configured to specifically generate laser pulses with an electrical pulse width at least 1000 $\mu$s.

In another embodiment, the method for fragmenting calculi comprises a source of laser pulses configured to specifically generate laser pulses with an electrical pulse width of 1000 $\mu$s.

In another embodiment, the method for fragmenting calculi comprises a source of laser pulses configured to specifically generate laser pulses with an electrical pulse width of 1250 $\mu$s.

Other systems, methods, features and advantages of the invention will be, or will become, apparent to one with skill in the art upon examination of the following figures and detailed description. It is intended that all such additional systems, methods, features, and advantages be within the scope of the invention, and be encompassed by the following claims.

## BRIEF DESCRIPTION OF THE DRAWINGS

The accompanying drawings are included to provide further understanding of the claims, are incorporated in, and constitute a part of this specification. The detailed description and illustrated examples described serve to explain the principles defined by the claims.

Figure 1 is a block diagram of an embodiment of the laser system used to generate the ideal values for laser parameters for calculi removal.

Figure 2 is a flow chart of an embodiment of the laser system used to generate the ideal values for laser parameters for calculi removal.

Figure 3 is a graph with electrical pulse width represented on the x-axis and optical pulse width represented on the y-axis wherein optical pulse width is shown as a function of electrical pulse length for a Ho:YAG laser.

Figure 4 is a graph with electrical pulse width represented on the x-axis and optical pulse width represented on the y-axis, wherein retropulsion length is shown as a function of electrical pulse width for a Ho:YAG laser using two different fiber sizes.

Figure 5 is a graph with electrical pulse width represented on the x-axis and crater volume represented on the y-axis, wherein crater volume is shown as a function of electrical pulse width for a Ho:YAG laser using two different fiber sizes.

Figure 6 is a graph with electrical pulse width represented on the x-axis and $\frac{retropulsion\ distance}{ablation\ volume}$ ("ideality) represented on the y-axis, wherein ideality is shown as a function of pulse width for a Ho:YAG laser using two different fiber sizes.

Figure 7 is a graph with electrical pulse energy represented on the x-axis and retropulsion length represented on the y-axis, wherein retropulsion length is shown as a function of electrical pulse energy for a Ho:YAG laser at two constant pulse widths.

Figure 8 is a graph with electrical pulse width represented on the x-axis and crater volume represented on the y-axis, wherein crater volume is shown as a function of electrical pulse energy for a Ho:YAG laser at two constant pulse widths.

Figure 9 is a graph with electrical pulse energy represented on the x-axis and $\frac{retropulsion\ distance}{ablation\ volume}$ ("ideality") represented on the y-axis, wherein ideality is shown as a function of electrical pulse energy for a Ho:YAG laser at two constant pulse widths.

## DETAILED DESCRIPTION OF THE INVENTION

Calculi can form in various parts of the body, such as the kidneys or the gallbladder, which can cause pain or damage to the body if not removed. Open surgical intervention was once the standard treatment for the removal of calculi, particularly when such calculi was located in a body lumen. However, less invasive techniques have emerged as safe and effective alternatives for the removal of calculi in the body.

Lithotripsy is a less invasive technique used to remove calculi in the body. It involves the crushing of the calculi into fragments that are easier to remove from the body. Lasers are often used as a power source for lithotripsy as the laser fiber is small and therefore the aperture of the working channel can be minimized. Laser systems may be used to break down calculi into smaller pieces. In particular, the laser system may be configured to generate and output a laser beam or other high concentrated beam of energy which may be transmitted to the treatment site. At the treatment site, the laser beam fragments, pulverizes or erodes the calculi.

Ho:YAG lasers may be used to break down calculi or stones into smaller pieces to facilitate removal of the calculi. The Ho:YAG laser can be used not only for the removal of calculi, but also for other soft tissue procedures. The Ho:YAG laser is typically transmitted through a fiber. When a Ho:YAG laser, after travelling the length of the fiber, is fired into a liquid medium the laser energy produces a vaporization bubble. The Ho:YAG laser produces a light at a wavelength of about 2.0 to 2.1 microns, depending on the precise formulation of the Ho:YAG rod, in a pulsed fashion. The Ho:YAG laser is effective because the aforementioned wavelengths are well absorbed by water and other liquid mediums. Further, all stones in a body lumen absorb this wavelength well, regardless of the stone color, because of the water in and on the surface of the stone. Although the various commercial models of Ho:YAG lasers vary slightly, the pulse duration of the Ho:YAG laser ranges from 250-350 μs, pulse energy from 0.2-4.0 J/pulse, frequency from 5-45 Hz and the average power from 30-80 W.

The dominant mechanism in Ho:YAG laser lithotripsy is photothermal along with the added minor effects of acoustic emission. Direct light absorption of the calculi increases the temperature of the irradiated volume above the ablation threshold, thereby causing the ejection of fragmented breakdown products. As well, absorption of laser energy by water between the stone and the fiber tip induces vapor bubble formation and collapse that generates shock waves. This laser-calculi interaction subjects the calculi to retropulsion forces induced by the combined effects of the ablated particle ejection, interstitial water vaporization, and bubble expansion and collapse. Therefore, the firing of each pulse causes the calculi to be displaced away from the fiber of the Ho:YAG laser.

Lasers rely on four main parameters for its performance - wavelength, spot size, pulse energy, and pulse width. As discussed earlier, the Ho:YAG laser produces a light at a wavelength of about 2.0 to 2.1 microns. The spot size of the laser is determined by the diameter of the fiber of the Ho:YAG laser, with a greater diameter achieving a greater spot size. Pulse energy determines the energy that the laser generates with each pulse. Pulse width, often referred to as pulse duration, can be quantified as either electrical pulse width or optical pulse width. Electrical pulse width is the time that the energy source to the laser is being pulsed. Optical pulse width is the time the laser light takes to exit the laser.

A clinical objective is to complete intracorporeal laser lithotripsy procedures as quickly as possible. To accomplish laser lithotripsy procedures quickly, experimental data has demonstrated that high pulse energy is better since it is faster at removing the calculi. However, retropulsion increases substantially at a higher pulse energy setting. Thus, a urologist might choose a high pulse energy setting to finish the case quickly as long as the potential need to chase the stone is acceptable. This strategy might be particularly useful for large bladder stones, for which stone volume requires lengthy operative time devoted to laser lithotripsy and the large stone mass has less retropulsion than a smaller stone. For instance, the use of high pulse energy would work be much more effective on large kidney and bladder stones that have a sizes that fall on the range between 1 - 2 sonometers.

Conversely not all urologists would accept this trade-off of faster lithotripsy but with greater retropulsion, especially for ureteroscopy. Retropulsion implies increased and potentially wasted operative time spent chasing the stone. Retropulsion also implies less lithotripsy since the high pulse energy settings produce more retropulsion and less fragmentation. Moreover, the larger fragments produced by high pulse energy may require basketing and increase costs due to increased operative time and basket use. A strategy of low pulse energy to create tiny debris may produce slow lithotripsy but potentially may not be as inefficient when compared to the time spent chasing the stone and its fragments as a result of retropulsion. Particularly, the use of high pulse energy would be less effective on smaller calculi, such as ureteral stones, that are often between 6-7 millimeters in size. In smaller calculi, the stones are prone to more movement and therefore a strategy of low pulse energy is more efficient as it provides for less operative time and basket use.

The present disclosure describes a laser system that is configured to operate at a predetermined set of laser parameter values to output a laser beam that removes calculi with minimum retropulsion. Effectiveness is maximized by minimizing the movement of the calculi. The present disclosure describes a set of laser parameter values that minimizes retropulsion by maximizing the pulse width of the laser.

The embodiments described in this disclosure will be discussed generally in relation to application of ideal values of laser parameters of a laser system to fragment smaller calculi in lithotripsy, but the disclosure is not so limited and may be applied to other soft tissue procedures, such as the removal of polyps and tumors.

As used herein, the term "retropulsion" refers to the amount of stone migration in the body of a human or mammal being acted upon by a laser pulse. High retropulsion can cause trauma to the tissue and potential ureter perforation.

The term "ablation" refers to the volume of stone that is removed from the surface of the calculi.

The term "ideality" refers to the measure of effectiveness of the laser system at fragmenting and removing smaller calculi in the body. Ideality is measured as:

$$Ideality = \frac{Retropulsion\ Distance\ (mm)}{Ablation\ Volume\ (mm^3)}$$

The parameters of the laser are more effective the lower the ideality ratio is - (e.g. higher ablation volume and lower retropulsion distance).

The term "ideal values" refer to values for laser parameters that are adapted to effectively fragment and remove smaller calculi within the body.

In one aspect, the ideal values for the laser parameters at which the laser system is operated are chosen to reduce retropulsion distance. This reduces or eliminates any trauma caused by chasing the stone in the body or using baskets and graspers to remove stone fragments. Ideal values for the laser parameters are achieved by reducing the peak power of the laser by increasing the electrical pulse width and reducing the electrical pulse energy.

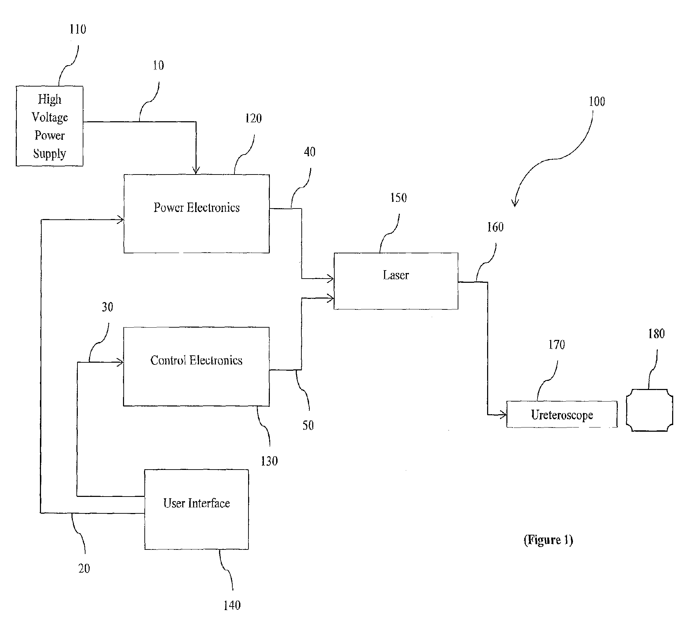

Figure 1 illustrates a block diagram of an example embodiment of a laser system **100** configured to operate in accordance with the ideal values for laser parameters for calculi removal.

The laser system **100** includes a high voltage power supply **110,** power electronics **120,** control electronics **130,** user interface **140,** a laser **150,** a fiber **160,** and an ureteroscope **170.** As shown in Fig. 1, the high voltage power supply **110** is connected through a high voltage connection **10** to the power electronics **120**. The user interface **140** is connected to the control electronics **130** through a data connection **30**. Similarly, the user interface **140** is connected to the power electronics **120** through a data connection **20**. The laser **150** is connected to the control electronics through a command data connection **50** and to the power electronics **120** through a high voltage connection **40**. The fiber **160** passes through the ureterorscope **170** at the distal end and the proximal face of the fiber **160** is connected to the laser **150**. The energy from laser **150** is transferred through the fiber **160** and the ureteroscope **170** before it is fired into the liquid medium at calculi **180**.

In operation, the laser system may be configured to operate in accordance with operating parameters to output a laser beam having corresponding characteristics to break down the calculi in a desired way. The ideal values for the laser parameters are entered or input into the user interface **140**. At least some of the laser parameters are variable parameters such as electrical pulse width, optical pulse width, pulse energy, and/or pulse frequency. Upon receipt of the ideal values, the user interface **140** sends the ideal values through the data connection **30** to the control electronics **130**. The control electronics **130** receives the ideal values from the user interface **140** and configures the laser parameters to output a laser with parameters having the ideal values. Similarly, the user interface **140** communicates with the power electronics **120** through the data connection **20** by sending the ideal values for laser parameters necessary to power the system. The power electronics **120** receives the signal from the user interface **140** and configures the laser parameter to the ideal value for power that will be generated when the laser is actuated.

In response to receipt of the ideal values from the user interface **140,** the control electronics **130** sends signals to the laser **150** through the command data connection **50** so that, when activated, the laser **150** emits a laser pulse having parameters that correspond with the ideal values input through the user interface **140**. The power electronics **120** provides the laser **150** with the amount of power indicated by the user interface **140** through the high voltage connection **40**. The high voltage power supply **110** supplies the power electronics **120** with the power to power the laser **150** through the high voltage connection **10**.

In further operation, the pulse generated from the laser **150** is transmitted through the fiber **160** to the treatment site where the calculi **180** is located. In one embodiment, the fiber **160** is a 365 $\mu$m fiber. In another embodiment, the fiber **160** is a 273 $\mu$m fiber.

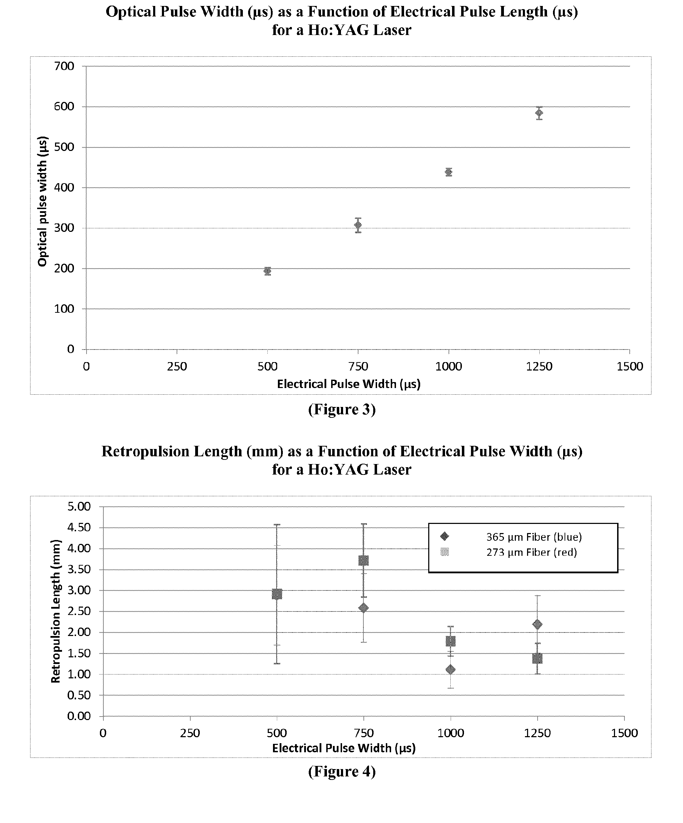

Figure 3 illustrates the relationship between electrical pulse width and optical pulse width for a Ho:YAG laser. An electrical pulse width of 1000 $\mu$s correlates with an optical pulse width of 438.8 $\mu$s and an electrical pulse width of 1250 $\mu$s correlates with an optical pulse width of 584.4 $\mu$s.

In one aspect of the disclosed parameters, the ideal electrical pulse width at which the laser will be operated correlates with an optical pulse width that is less than 1000 $\mu$s. This is because the accepted maximum thermal relaxation time for tissue is 1000 $\mu$s. Any tissue exposure to energy longer than this maximum thermal relaxation time allows heat to build up in the tissue and results in unintended thermal effects such as increased coagulation within the tissue.

In another aspect of the disclosed parameters, the ideal electrical pulse widths at which the laser will be operated at are 1000 $\mu$s and 1250 $\mu$s. In Figures 4-6, a conventional Ho:YAG laser was tested with a 365 $\mu$m fiber (blue) and a 273 $\mu$m fiber (red) at a 1 J per pulse energy through a range of electrical pulse widths from 500 $\mu$s to 1250 $\mu$s. The data points demonstrate an effective electrical pulse width at 1000 $\mu$s and 1250 $\mu$s.

In another aspect of the disclosed parameters, the ideal optical pulse widths at which the laser will be operated at are 438.8 $\mu$s and 584.4 $\mu$s.

Figure 4 illustrates the relationship between electrical pulse width and retropulsion length as the electrical pulse width is increased from 500 $\mu$s through 1250 $\mu$s. The retropulsion length increases up until an electrical pulse width of 750 $\mu$s and then decreases thereafter.

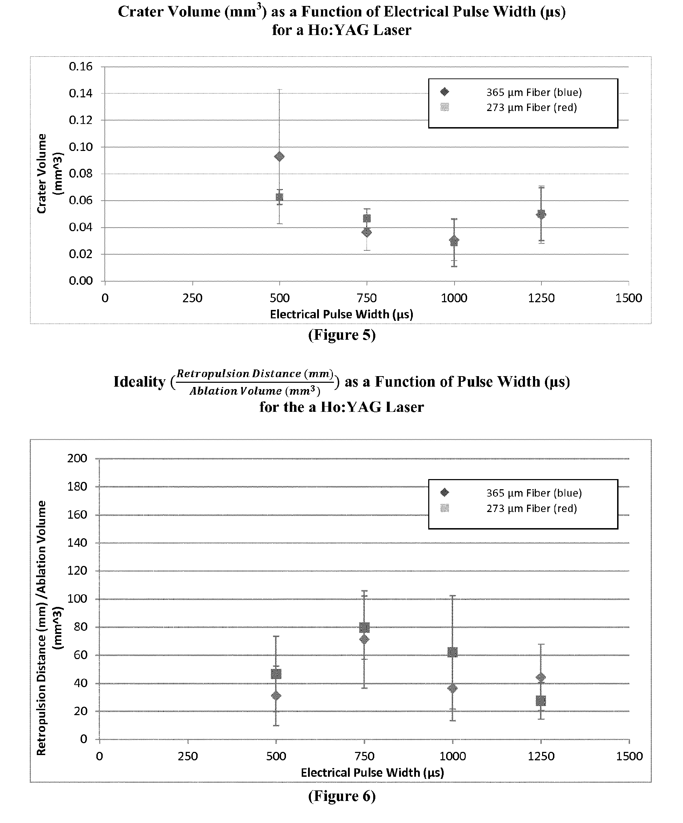

Figure 5 illustrates the relationship between electrical pulse width and crater volume (ablation) as the electrical pulse width is increased from 500 $\mu$s through 1250 $\mu$s. Ablation decreases up until an electrical pulse width of 1000 $\mu$s and increases thereafter.

Figure 6 illustrates the relationship between electrical pulse width and ideality as the electrical pulse width is increased from 500 $\mu$s through 1250 $\mu$s. Ideality increases through an electrical pulse width of 750 $\mu$s and decreases thereafter. Ideality is at its lowest at 1250 $\mu$s.

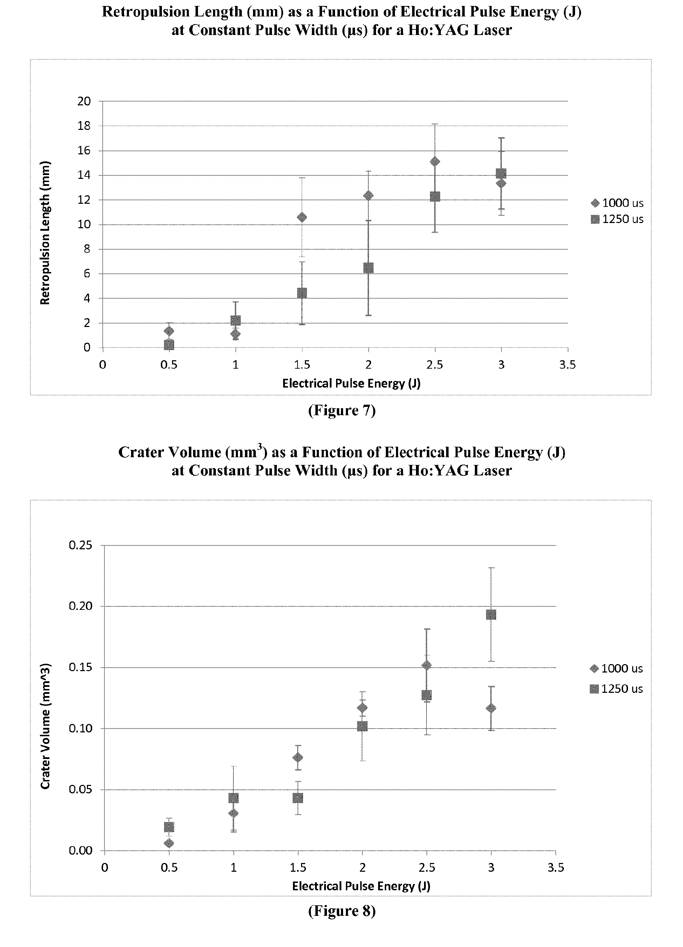

Figures 7-9 demonstrate the effect that increasing pulse energy has on retropulsion and ablation. In Figure 7-9, a Ho:YAG laser was tested at both an electrical pulse width of 1000 $\mu$s and 1250 $\mu$s. While keeping the electrical pulse width constant at either of these two values, the range of electrical pulse energies was increased in increments of 0.5 J from 0.5 J to 3.0 J.

Figure 7 illustrates the relationship between electrical pulse energy and retropulsion length as the electrical pulse energy was increased from 0.5 J through 3.0 J. The retropulsion length increased as electrical pulse energy was increased.

Figure 8 illustrates the relationship between electrical pulse energy and crater volume (ablation) as the electrical pulse energy is increased from 0.5 J through 3.0 J. The crater volume increased as electrical pulse energy increased through 3.0 J.

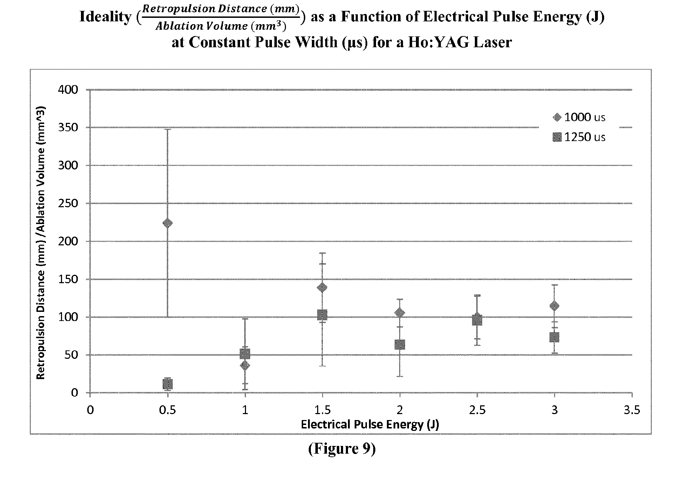

Figure 9 illustrates the relationship between electrical pulse energy and ideality as the electrical pulse energy is increased from 0.5 J through 3.0 J. Ideality increased as electrical pulse energy increased and was lowest at an electrical pulse energy of 0.5 J. Although, as seen in Figure 6, the crater volume increased as electrical pulse energy increased, the magnitude of increase in retropulsion length at higher electrical pulse energy rates offset the increase in crater volume as electrical pulse energy increased.

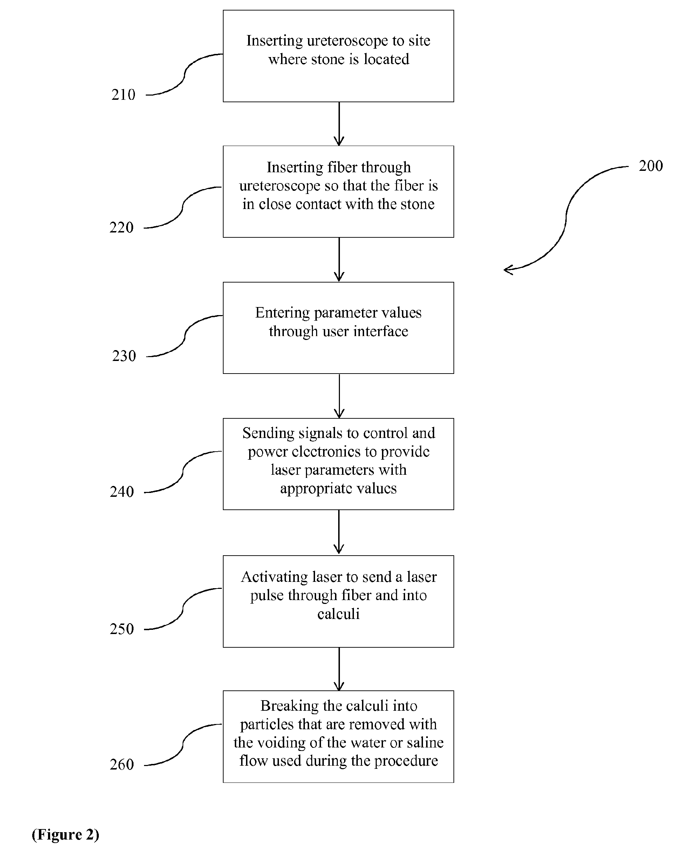

Figure 2 is a flow chart of an embodiment of a laser system **200** configured to determine and identify the ideal values for laser parameters for calculi removal. First, at block **210** of Figure 2, the ureteroscope **170** of Figure 1 is inserted into the body to the site where the calculi **180** is located.

Next, at block **220** of Figure 2, the fiber **160** of Figure 1 is inserted through the ureteroscope **170** so that the fiber is near the calculi **180,** with at least a film of water between the surface of the fiber **160** and the calculi **180.**

Next, at block **230** of Figure 2, parameter values are entered into user interface **140** of Figure 1 to attain ideal values for electrical pulse width or optical pulse. In one embodiment, the electric pulse width would be set at either 1000 $\mu$s or 1250 $\mu$s. In another embodiment, the optical pulse width would be set at either 438.8 $\mu$s or 584.4 $\mu$s.

Next, at block **240** of Figure 2, the user interface **140** of Figure 1 sends signals to the control electronics **130** and power electronics **120** to provide the ideal values for the laser parameters necessary to operate the laser **150.**

Next, at block **250** of Figure 2, the laser **150** of Figure 1 is activated to send a laser pulse through the fiber **160** and into the calculi **180.**

Next, at block **260** of Figure 2, the calculi **180** is broken into fine particles that are voided with the water or saline flow used during the procedure.

While particular elements, embodiments, and applications of the present invention have been shown and described, it is understood that the invention is not limited thereto because modifications may be made by those skilled in the art, particularly in light of the foregoing teaching. It is therefore contemplated by the appended claims to cover such modifications and incorporate those features which come within the scope of the invention. The described apparatus may be used exvivo for testing, development, training, demonstration or the like

**Claims**

1.   An apparatus for fragmenting calculi comprising:

a source of laser pulses,
an optical fiber having a distal end configured to be in close proximity with said calculi, and a proximal end that is configured to receive laser pulses from said source of laser pulses when said optical fiber is operatively engaged with said source of laser pulses, and
said source of laser pulses is configured to specifically generate laser pulses with an optical pulse width between 400 $\mu$s and 600 $\mu$s; an optical pulse width between 600 $\mu$s and 1000 $\mu$s; an electrical pulse width of at least 1000 $\mu$s or an electrical pulse width between 1000 $\mu$s and 1250 $\mu$s.

2.   The apparatus of claim 1 wherein said optical fiber used has a 365 $\mu$m or 273 $\mu$m fiber.

3.   The apparatus of any one of the preceding claims wherein said source of laser pulses is a Ho:YAG laser.

4.   The apparatus of any one of the preceding claims wherein said laser pulse has an optical pulse width of about 440 $\mu$s; 439 $\mu$s or 438.8 $\mu$s.

5.   The apparatus of any one of claim 1 to claim 3 wherein said laser pulse has an optical pulse width of about 580 $\mu$s; 585 $\mu$s or 584.4 $\mu$s.

6.   The apparatus of any one of the preceding claims wherein said laser pulse has an electrical pulse width of about 1000 $\mu$s or 1000 $\mu$s.

7.   The apparatus of any one of claim 1 to claim 5 wherein said laser pulse has an electrical pulse width of about 1250 $\mu$s or 1250 $\mu$s.

8.   A method for generating laser pulses having parameters effective in fragmenting calculi, the method comprising:

providing a source of laser pulses,
providing an optical fiber having a distal end configurable to be in close proximity with said calculi, and a proximal end that is configured to receive laser pulses from said source of laser pulses when said optical fiber is operatively engaged with said source of laser pulses, and

calibrating said source of laser pulses to specifically generate laser pulses with an optical pulse width between 400 $\mu$s and 600 $\mu$s; an optical pulse width between 600 $\mu$s and 1000 $\mu$s; an electrical pulse width of at least 1000 $\mu$s or an electrical pulse width between 1000 $\mu$s and 1250 $\mu$s.

9. The method of claim 8 wherein said optical fiber used has a 365 $\mu$m or 273 $\mu$m fiber.

10. The method of claim 8 or claim 9 wherein said source of laser pulses is a Ho:YAG laser.

11. The method of any one of claim 8 to claim 10 wherein said laser pulse has an optical pulse width of about 440 $\mu$s; 439 $\mu$s or 438.8 $\mu$s.

12. The method of any one of claim 8 to claim 10 wherein said laser pulse has an optical pulse width of about 580 $\mu$s; 585 $\mu$s or 584.4 $\mu$s.

13. The method of any one of claim 8 to claim 12 wherein said laser pulse has an electrical pulse width of about 1000 $\mu$s or 1000 $\mu$s.

14. The method of any one of claim 8 to claim12 wherein said laser pulse has an electrical pulse width of about 1250 $\mu$s or 1250 $\mu$s.

(Figure 1)

210 → Inserting ureteroscope to site where stone is located

220 → Inserting fiber through ureteroscope so that the fiber is in close contact with the stone

230 → Entering parameter values through user interface

240 → Sending signals to control and power electronics to provide laser parameters with appropriate values

250 → Activating laser to send a laser pulse through fiber and into calculi

260 → Breaking the calculi into particles that are removed with the voiding of the water or saline flow used during the procedure

200

**(Figure 2)**

**Optical Pulse Width (µs) as a Function of Electrical Pulse Length (µs)
for a Ho:YAG Laser**

**(Figure 3)**

**Retropulsion Length (mm) as a Function of Electrical Pulse Width (µs)
for a Ho:YAG Laser**

**(Figure 4)**

**Crater Volume (mm$^3$) as a Function of Electrical Pulse Width (µs)
for a Ho:YAG Laser**

(Figure 5)

**Ideality ($\frac{Retropulsion\ Distance\ (mm)}{Ablation\ Volume\ (mm^3)}$) as a Function of Pulse Width (µs)
for the a Ho:YAG Laser**

(Figure 6)

**Retropulsion Length (mm) as a Function of Electrical Pulse Energy (J)
at Constant Pulse Width (µs) for a Ho:YAG Laser**

(Figure 7)

**Crater Volume (mm$^3$) as a Function of Electrical Pulse Energy (J)
at Constant Pulse Width (µs) for a Ho:YAG Laser**

(Figure 8)

**Ideality ($\frac{Retropulsion\ Distance\ (mm)}{Ablation\ Volume\ (mm^3)}$) as a Function of Electrical Pulse Energy (J) at Constant Pulse Width (µs) for a Ho:YAG Laser**

(Figure 9)

Europäisches Patentamt
European Patent Office
Office européen des brevets

## EUROPEAN SEARCH REPORT

Application Number

EP 15 16 6049

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | "SWISS LASERCLAST, New universal Holium:YAG laser system", ， 1 August 2012 (2012-08-01), XP055209014, Retrieved from the Internet: URL:http://www.ems-company.com/media/PDF NEW/FA-398_EN_Ed_08-2012_LaserClast Brochure_single.pdf [retrieved on 2015-08-21] * the whole document * | 1-7 | INV. A61B18/26 A61B18/24 |
| X | Rocamed: "Laser", ， 1 September 2013 (2013-09-01), XP055209020, Retrieved from the Internet: URL:http://www.rocamed-urology.com/images/ stories/doc/br.uk.v2.laser.pdf [retrieved on 2015-08-21] * the whole document * | 1-7 | |
| X | PATEL ABHISHEK P ET AL: "Optimizing Use of the Holmium:YAG Laser for Surgical Management of Urinary Lithiasis", CURRENT UROLOGY REPORTS, CURRENT SCIENCE, US, vol. 15, no. 4, 15 February 2014 (2014-02-15), pages 1-7, XP035375591, ISSN: 1527-2737, DOI: 10.1007/S11934-014-0397-2 [retrieved on 2014-02-15] * the whole document * | 1-7 | TECHNICAL FIELDS SEARCHED (IPC) A61B |

-/--

The present search report has been drawn up for all claims

| Place of search | Date of completion of the search | Examiner |
|---|---|---|
| Munich | 21 August 2015 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 15 16 6049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FELIX WEZEL ET AL: "Effect of Pulse Energy, Frequency and Length on Holmium:Yttrium-Aluminum-Garnet Laser Fragmentation Efficiency in Non-Floating Artificial Urinary Calculi", JOURNAL OF ENDOUROLOGY, vol. 24, no. 7, 1 July 2010 (2010-07-01), pages 1135-1140, XP055208957, ISSN: 0892-7790, DOI: 10.1089/end.2010.0115 * abstract * | 1-3 | |
| X | PANKAJ KALRA ET AL: "Effect of Pulse Width on Object Movement In Vitro Using Holmium:YAG Laser", JOURNAL OF ENDOUROLOGY, vol. 21, no. 2, 1 February 2007 (2007-02-01), pages 228-231, XP055209011, ISSN: 0892-7790, DOI: 10.1089/end.2005.1130 * abstract * | 1,3 | |
| X | David S Finley ET AL: "Effect of holmium:YAG laser pulse width on lithotripsy retropulsion in vitro", Journal of endourology / Endourological Society, 1 October 2005 (2005-10-01), pages 1041-1044, XP055209029, United States DOI: 10.1089/end.2005.19.1041 Retrieved from the Internet: URL:http://www.ncbi.nlm.nih.gov/pubmed/16253078 [retrieved on 2015-08-21] * abstract * | 1,3 | TECHNICAL FIELDS SEARCHED (IPC) |

-/--

The present search report has been drawn up for all claims

| Place of search | Date of completion of the search | Examiner |
|---|---|---|
| Munich | 21 August 2015 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 15 16 6049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 820 627 A (ROSEN DAVID I [US] ET AL) 13 October 1998 (1998-10-13) * abstract; claims 1,6,10 * * column 3, line 51 - line 60 * ----- | 1,3,4,6 | |
| X | WO 00/48525 A2 (SCIMED LIFE SYSTEMS INC [US]) 24 August 2000 (2000-08-24) * abstract * * page 9, line 4 - page 10, line 12 * ----- | 1-3 | |
| X | US 2013/218146 A1 (HENNINGS DAVID R [US] ET AL) 22 August 2013 (2013-08-22) * paragraph [0167] - paragraph [0170] * ----- | 1,2 | |
| X | EP 2 596 759 A1 (TECHLAMED S R L SOCIETA UNIPERSONALE [IT]) 29 May 2013 (2013-05-29) * paragraph [0013] - paragraph [0016] * * paragraph [0022] * ----- | 1,3-7 | |
| X | WO 92/08515 A2 (BARD INC C R [US]) 29 May 1992 (1992-05-29) * abstract; figure 8 * * page 29, line 12 - page 33, line 5 * ----- | 1,3 | TECHNICAL FIELDS SEARCHED (IPC) |

The present search report has been drawn up for all claims

| Place of search | Date of completion of the search | Examiner |
|---|---|---|
| Munich | 21 August 2015 | Beck, Ewa |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

&  : member of the same patent family, corresponding
     document

EPO FORM 1503 03.82 (P04C01)

**EP 2 939 631 A1**

**ANNEX TO THE EUROPEAN SEARCH REPORT
ON EUROPEAN PATENT APPLICATION NO.**

EP 15 16 6049

This annex lists the patent family members relating to the patent documents cited in the above-mentioned European search report.
The members are as contained in the European Patent Office EDP file on
The European Patent Office is in no way liable for these particulars which are merely given for the purpose of information.

21-08-2015

| Patent document cited in search report | | Publication date | Patent family member(s) | | Publication date |
|---|---|---|---|---|---|
| US 5820627 | A | 13-10-1998 | NONE | | |
| WO 0048525 | A2 | 24-08-2000 | AU | 3700700 A | 04-09-2000 |
| | | | CA | 2362332 A1 | 24-08-2000 |
| | | | DE | 60024585 T2 | 03-08-2006 |
| | | | EP | 1154727 A2 | 21-11-2001 |
| | | | JP | 4310049 B2 | 05-08-2009 |
| | | | JP | 2002537017 A | 05-11-2002 |
| | | | US | 2002002366 A1 | 03-01-2002 |
| | | | US | 2002103477 A1 | 01-08-2002 |
| | | | US | 2004243123 A1 | 02-12-2004 |
| | | | WO | 0048525 A2 | 24-08-2000 |
| US 2013218146 | A1 | 22-08-2013 | NONE | | |
| EP 2596759 | A1 | 29-05-2013 | EP | 2596759 A1 | 29-05-2013 |
| | | | US | 2013138033 A1 | 30-05-2013 |
| WO 9208515 | A2 | 29-05-1992 | US | 5275594 A | 04-01-1994 |
| | | | WO | 9208515 A2 | 29-05-1992 |

EPO FORM P0459

For more details about this annex : see Official Journal of the European Patent Office, No. 12/82